# DEMANDE DE BREVET EUROPEEN

(11) **EP 1 559 392 A1**
(43) Date de publication de la demande: **03.08.2005**
(21) Numéro de dépôt: 05290176.6
(22) Date de dépôt: 27.01.2005
(51) Int. Cl.: A61K 7/027, A61K 7/032, A61K 7/043, A61K 7/42

(54) **Procédé de préparation d'une composition pour la coloration permanente ou semi-permanente de la peau, des paupières, des lèvres ou des ongles à partir de fluide sous pression et d'agents de coloration**

(30) Priorité: 29.01.2004 FR 0400857
(71) Demandeur: L'OREAL, 75008 Paris (FR)
(72) Inventeur: Grollier, Jean-François, 75006 Paris (FR)
(74) Mandataire: Casalonga, Axel

(57) **Abrégé**

L'invention concerne un procédé de préparation d'une composition de coloration permanente ou semi-permanente de la peau, des paupières, des lèvres ou des ongles, comprenant une étape de percolation de fluide sous une pression d'au moins 3 bars au travers d'au moins un agent de coloration sous forme solide ou pâteuse. Elle concerne aussi un procédé de coloration permanente ou semi-permanente de la peau, des paupières, des lèvres ou des ongles mettant en oeuvre la composition de coloration obtenue selon le procédé de l'invention.

## Description

La présente invention a pour objet un procédé de préparation d'une composition de coloration permanente ou semi-permanente de la peau, des paupières, des lèvres ou des ongles, ainsi qu'un procédé de coloration permanente ou semi-permanente de la peau, des paupières, des lèvres ou des ongles à partir de cette composition.

Les produits de maquillage traditionnels tels que, par exemple, les fards à joues, les fards à paupières, les fonds de teint, les anti-cernes ou les bâtons à lèvres, contiennent généralement des pigments. Ils permettent d'améliorer l'esthétique en conférant plus de relief au visage, aux yeux et aux lèvres, et en intensifiant leur couleur.

Ces produits sont couvrants et ont en général un effet momentané, c'est-à-dire qu'ils s'éliminent facilement au moyen d'eau ou d'une solution contenant des tensioactifs.

De nos jours, on recherche de plus en plus des produits de coloration permanente ou semi-permanente, par exemple pour donner à la peau une couleur proche de celle d'un bronzage naturel, pour maquiller les ongles de manière plus durable, pour souligner le contour des yeux et des lèvres de manière plus prolongée, et également pour réaliser des tatouages. Les formulations utilisées à ce propos permettent notamment d'avoir bonne mine et d'être maquillé pendant plusieurs jours sans avoir à se maquiller tous les jours.

Au sens de la présente invention, on entend par «composition de coloration permanente ou semi-permanente», toute composition ayant une affinité particulière pour la peau, les paupières, les lèvres ou les ongles lui permettant de conférer à la peau, aux paupières, aux lèvres ou aux ongles une coloration non couvrante (qui n'opacifie pas) et durable, qui ne s'élimine pas facilement à l'eau ni à l'aide d'un solvant, et qui résiste à la fois au frottement et au lavage par une solution contenant des tensioactifs. Une telle coloration durable se distingue donc de la coloration superficielle, couvrante et momentanée apportée par exemple par un produit de maquillage classique.

Au sens de la présente invention, on entend par «agent de coloration», toute substance ayant une affinité particulière pour la peau, les paupières, les lèvres ou les ongles lui permettant de conférer à la peau, aux paupières, aux lèvres ou aux ongles une coloration durable telle que définie précédemment.

Cependant, certains agents de coloration présentent l'inconvénient d'être instables vis-à-vis de l'eau, d'agents oxydants ou de la lumière, à savoir ils subissent une dégradation par un mécanisme d'hydrolyse, d'oxydation, de photolyse ou photodégradation. Le phénomène d'oxydation peut avoir plusieurs causes, en particulier la présence d'eau, d'agents oxydants comme l'oxygène de l'air, ou de lumière et notamment du rayonnement ultraviolet.

Ces agents de coloration peuvent également présenter l'inconvénient de réagir avec d'autres actifs cosmétiques utilisés couramment en cosmétique, lorsqu'ils sont utilisés en solution aqueuse.

En outre, les compositions de coloration permanente ou semi-permanente de la peau, des paupières, des lèvres ou des ongles, au sens de la présente invention, sont généralement des compositions aqueuses dans lesquelles lesdits agents de coloration doivent être solubilisés. Le manque de solubilité de ces composés diminue le pouvoir colorant de ces compositions. Ce critère de solubilité peut réduire le nombre d'agents de coloration que l'on peut utiliser pour la coloration de la peau, des paupières, des lèvres ou des ongles. Ceci est particulièrement le cas des composés à point de fusion élevé.

La Demanderesse a découvert de manière surprenante qu'en utilisant un nouveau procédé de préparation d'une composition de coloration permanente ou semi-permanente de la peau, des paupières, des lèvres ou des ongles, on pouvait obtenir en un temps très court, inférieur à 2 minutes, des compositions plus ou moins concentrées en agent(s) de coloration selon le besoin, notamment sans conservateur, permettant de surmonter les inconvénients exposés ci-dessus.

Ce procédé est mis en oeuvre simplement et est approprié au besoin du consommateur. On fait passer un fluide sous pression, dont la température est de préférence supérieure ou égale à 30 °C, pendant un laps de temps très court, à travers au moins un agent de coloration sous forme solide ou pâteuse, de préférence solide, et encore plus préférentiellement pulvérulente.

Il permet ainsi d'utiliser sous forme anhydre des agents de coloration instables dans des compositions aqueuses, soit parce qu'ils réagissent avec l'eau ou avec l'humidité ambiante, soit parce qu'ils réagissent en solution aqueuse avec des composés qui ne réagissent pas avec eux dans une composition anhydre, et de les stocker sans risque de dégradation.

Les compositions préparées selon ce procédé peuvent présenter une stabilité au stockage limitée, ce qui n'est pas ici un inconvénient puisque le procédé conduit à une composition prête-à-l'emploi, destinée à être utilisée rapidement après sa préparation, par exemple dans les 5 minutes suivant sa préparation, notamment après refroidissement à une température acceptable pour les matières kératiniques, de préférence inférieure à 60 °C, mieux inférieure à 50°C. La composition peut être également utilisée jusqu'à une semaine après sa préparation, selon la vitesse de dégradation de l'agent de coloration utilisé.

Etant donné le temps de préparation très court, les compositions de coloration permanente ou semi-permanente de la peau, des paupières, des lèvres ou des ongles peuvent être préparées « à la demande » en mélangeant les agents de coloration, i.e. selon la couleur recherchée, dans un mode particulier de réalisation.

Selon un autre mode de réalisation, les agents de coloration pouvant être conditionnés dans un dispositif prêt-à-l'emploi, il n'est pas nécessaire de déterminer au préalable les concentrations desdits agents en solution, ce qui limite les erreurs de mesure de l'utilisateur.

En outre, le procédé selon l'invention permet d'éviter l'utilisation de flacons multi-compartiments, ce qui rend le procédé particulièrement économique et plus sûr pour l'utilisateur.

La composition ainsi obtenue peut être utilisée seule ou en mélange avec une autre composition.

L'invention a donc pour objet un procédé de préparation d'une composition de coloration permanente ou semi-permanente de la peau, des paupières, des lèvres ou des ongles comprenant une étape de percolation d'un fluide sous une pression d'au moins 3 bars (3.10⁵ Pa) au travers d'au moins un agent de coloration sous forme solide ou pâteuse.

Un autre objet de l'invention est une composition susceptible d'être obtenue par le procédé selon l'invention.

L'invention a également pour objet l'utilisation de la composition obtenue selon le procédé de l'invention pour la coloration permanente ou semi-permanente de la peau, des paupières, des lèvres ou des ongles et de préférence de la peau, des paupières ou des lèvres.

Un autre objet de l'invention est un procédé de coloration permanente ou semi-permanente de la peau, des paupières, des lèvres ou des ongles mettant en oeuvre la composition de coloration permanente ou semi-permanente de la peau, des paupières, des lèvres ou des ongles obtenue selon le procédé de l'invention.

L'invention a encore pour objet un dispositif de conditionnement permettant de mettre en oeuvre le procédé de préparation de la présente invention.

D'autres objets, caractéristiques, aspects et avantages de l'invention apparaîtront encore plus clairement à la lecture de la description et des exemples qui suivent.

Selon l'invention, le procédé de préparation d'une composition de coloration permanente ou semi-permanente de la peau, des paupières, des lèvres ou des ongles comprend une étape de percolation d'un fluide de préférence à une température supérieure ou égale à 30 °C, mieux encore allant de 30 à 150 °C, et encore plus préférentiellement de 40 à 120 °C, sous une pression d'au moins 3 bars (3. 10⁵ Pa) au travers d'au moins un agent de coloration sous forme solide ou pâteuse.

La percolation est un mouvement de fluide à travers un milieu poreux permettant le passage du fluide, sous l'action ou l'effet de la pression.

Le fluide peut être constitué par de la vapeur d'eau éventuellement accompagnée d'eau liquide, ou par un ou plusieurs solvants liquides et/ou gazeux cosmétiquement acceptables, ou encore par un mélange de vapeur d'eau éventuellement accompagnée d'eau liquide, et d'un ou plusieurs solvants liquides et/ou gazeux cosmétiquement acceptables. De préférence, le fluide comprend au moins de la vapeur d'eau pouvant être accompagnée d'eau liquide, et encore plus préférentiellement il est de la vapeur d'eau pouvant être accompagnée d'eau liquide.

A titre de solvant organique, on peut citer, par exemple, les alcools inférieurs en C₁-C₄ tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

L'agent de coloration est sous forme solide ou pâteuse, de préférence sous forme solide, et encore plus préférentiellement pulvérulente.

Par « forme pâteuse » au sens de la présente invention, on entend une consistance intermédiaire entre une phase solide et une phase liquide. La viscosité de cette phase pâteuse est de préférence supérieure ou égale à 0,1 Pa.s, et encore plus préférentiellement supérieure ou égale à 1 Pa.s, ceci à 25°C avec un taux de cisaillement de 10 s⁻¹.

Le procédé de la présente invention peut être mis en oeuvre à partir d'un dispositif classique permettant de générer un fluide sous pression, à une température de préférence supérieure ou égale à 30 °C, mieux encore allant de 30 à 150 °C, et encore plus préférentiellement de 40 à 120 °C. Un tel dispositif comprend une chambre résistant à la pression, équipée d'un bloc thermique, ainsi qu'un circuit d'acheminement du fluide produit vers l'agent de coloration utilisé dans l'invention.

Selon un autre mode de réalisation, le dispositif comprend en outre un réservoir de liquide(s) ainsi qu'une pompe permettant l'acheminement du ou des liquides vers la chambre.

Le liquide contenu dans le réservoir est soit de l'eau, soit un solvant cosmétiquement acceptable ou un mélange de plusieurs solvants cosmétiquement acceptables, ou encore un mélange d'eau et d'un ou plusieurs solvants cosmétiquement acceptables. De préférence, le liquide comprend au moins de l'eau, et encore plus préférentiellement il est de l'eau.

Un dispositif particulièrement utile pour la mise en oeuvre du procédé de la présente invention est une machine à café du type « expresso ». De telles machines sont bien connues de la technique. Par exemple, ces machines sont décrites dans les brevets AT 168405, US 2688911, DE 32433870 et IT 1265636.

Selon un mode de réalisation particulier de l'invention, l'étape de percolation est mise en oeuvre avec un fluide à une température de préférence supérieure ou égale à 30 °C, mieux encore allant de 30 à 150 °C, sous une pression de 3 à 30 bars (3.10⁵ et 3. 10⁶ Pa), de préférence d'au moins 4 bars (4. 10⁵ Pa), mieux encore supérieure ou égale à 10 bars (10⁶ Pa) et tout particulièrement de 10 à 30 bars (10⁶ et 3. 10⁶ Pa).

Une composition cosmétique comprenant au moins un agent de coloration sous forme solide ou pâteuse, peut être utilisée directement dans le dispositif générant le fluide sous pression dans un récipient destiné à cet usage. Elle peut également être conditionnée dans un dispositif de conditionnement particulier comprenant un logement fermé délimité par au moins une paroi au moins en partie perméable au fluide sous une pression d'au moins 3 bars (3. 10⁵ Pa). De tels dispositifs sont, par exemple, décrits dans les demandes de brevets WO 00/56629, EP512470, US5897899 ou WO 99/03573. Ces dispositifs de conditionnement sont en général étanches à l'air, l'humidité et/ou la lumière.

Selon un mode de réalisation particulier, le logement est délimité par deux feuilles scellées. Selon un autre mode de réalisation, le logement est délimité par une barquette fermée par un couvercle.

Ces dispositifs peuvent être fabriqués à partir de matériaux tissés ou non tissés, en matière plastique ou végétale, par exemple, en cellulose, en métal tel que l'aluminium ou en composite. De tels dispositifs sont par exemple décrits dans les demandes de brevets WO 00/56629, EP 512470, US5897899 ou WO 99/03573.

Les agents de coloration utilisés dans l'invention sont notamment choisis parmi les autobronzants, les composés indoliques, les extraits végétaux colorants, les sels de flavylium non substitués en position 3 et substitués par au moins un groupe hydroxy ou alcoxy, les précurseurs de colorant du type polyphénol, les colorants naturels, le carmin de cochenille, les fluoranes ou leurs sels de métal alcalin, les activateurs de la mélanogénèse, et leurs mélanges.

A titre d'exemples d'autobronzants, on peut notamment citer les composés mono- ou polycarbonylés. Les agents autobronzants mono ou polycarbonylés peuvent être choisis, par exemple, parmi l'isatine, l'alloxane, la ninhydrine, le glycéraldéhyde, l'aldéhyde mésotartrique, la glutaraldéhyde, l'érythrulose, les dérivés de pyrazolin-4,5-diones telles que décrits dans la demande de brevet FR 2466492 et WO 97/35842, la dihydroxyacétone (DHA), les dérivés de 4,4-dihydroxypyrazolin-5-ones tels que décrits dans la demande de brevet EP 0903342.

Dans un mode de réalisation particulièrement préféré de l'invention, on utilisera plus particulièrement la dihydroxyacétone (DHA) comme agent autobronzant.

Comme composés indoliques, on peut notamment citer les monohydroxyindoles tels que décrits dans le brevet FR 2651126 ou les dihydroxyindoles tels que décrits dans le brevet EP 425324. Des exemples particuliers de ces composés sont les 4-, 5-, 6- ou 7-hydroxyindoles, les 5,6-dihydroxyindole, 2-méthyl-5,6-dihydroxyindole, 3-méthyl-5,6-dihydroxyindole et 2,3-diméthyl-5,6-dihydroxyindole, et plus préférentiellement les 5- et 6- hydroxyindoles, et la 5,6-dihydroxyindole, et encore plus préférentiellement la 5,6-dihydroxyindole.

A titre d'extraits végétaux colorants, on peut citer par exemple :
- les extraits végétaux du genre Sorgho comme le Sorghum caudatum, le Sorghum Bicolor tels que décrits dans les demandes EP 1172090 et EP 1327437.

Les sels de flavylium non substitués en position 3 et substitués par au moins un groupe hydroxy ou alcoxy, sont obtenus par voie de synthèse ou à partir d'un extrait végétal les contenant ou bien encore à partir d'un extrait enrichi. Ces sels sont notamment décrits dans la demande EP1172090 et des exemples préférés de ces sels sont les chlorures des composés suivants :
- 4', 5, 7-trihydroxyflavylium, communément dénommé "chlorure d'apigéninidine",
- 3', 4', 7- trihydroxyflavylium,
- 4'-hydroxyflavylium,
- 4', 7-dihydroxyflavylium,
- 3', 4'-dihydroxyflavylium,
- 3', 4'-dihydroxy-7-méthoxy-flavylium,
- 3',4', 5, 7-tétrahydroxyflavylium,
- 3', 4', 5', 5, 7-pentahydroxyflavylium.

Parmi ces composés, le chlorure d'apigéninidine (chlorure de 4', 5, 7-trihydroxyflavylium) et le chlorure de 3', 4', 7-trihydroxyflavylium sont encore plus particulièrement préférés.

Le chlorure d'apigéninidine peut être également utilisé sous forme d'un extrait végétal, aisément préparé par extraction, et isolé, à partir de feuilles de Sorghum caudatum selon les procédés décrits dans les brevets CN 1064284A et CN1035512C ou toutes autres variantes de ces procédés.

Comme précurseurs de colorant du type polyphénol (i.e. orthodiphénol), on peut utiliser dans le procédé de l'invention, ceux décrits dans la demande EP 1229892. Les précurseurs de colorant du type polyphénol préférés sont :
- les flavanols comme la catéchine et le gallate d'épichatéchine,
- les flavonols comme la quercétine,
- les anthocyanidines comme la péonidine,
- les anthocyanines, par exemple l'oenine,
- les hydroxybenzoates, par exemple l'acide gallique,
- les flavones comme la lutéoline,
- les iridoïdes comme l'oleuropéine,
ces produits pouvant être osylés (par exemple glucosylés) et /ou sous forme d'oligomères (procyanidines) ;
- les hydroxystilbènes, par exemple le 3,3',4,5'-tétrahydroxystilbène, éventuellement osylés (par exemple glucosylés) ;
- la 3,4-dihydroxyphénylalanine et ses dérivés ;
- la 2,3-dihydroxyphénylalanine et ses dérivés ;
- la 4,5-dihydroxyphénylalanine et ses dérivés ;
- les dihydroxycinnnamates tels que l'acide caféique et l'acide chlorogénique ;
- les hydroxycoumarines ;
- les hydroxyisocoumarines ;
- les hydroxycoumarones;
- les hydroxyisocoumarones;
- les hydroxychalcones ;
- les hydroxychromones ;
- les anthocyanes ;
- les quinones ; et
- les hydroxyxanthones.

A titre de colorants naturels, on peut citer par exemple :
- les flavonoïdes et leurs esters, éthers, hétérosides et polymères
- les polyphénols non flavonoïdes et leurs esters, éthers, hétérosides et polymères ,
- les caroténoïdes tels que les β-carotènes, et les β-laïnes,
- les chlorophylles ;
- les hydrates de carbone (caramel) ;
- les mélanines, par exemple les eumélanines et les phéomélanines.

Le carmin de cochenille utilisable dans le procédé de l'invention est celui notamment décrit dans la demande WO 02/41867. Il est également connu sous notamment les noms suivants : CI 75470, Natural Red 4, Carminic Acid, Carmine 5297, Carmine Ultra-Fine, Carminic Acid Lake , E120.

Les colorants rouges ou oranges utilisables dans le procédé de l'invention sont notamment choisis parmi les fluoranes et leurs sels de métal alcalin tels que décrits dans la demande FR 2840806. Plus particulièrement, on peut utiliser la tétrabromofluoroscéine ou éosine connu sous le nom CTFA CI 45380 ou Red 21 ; la phloxine B connu sous le nom CTFA CI 45410 ou Red 27 ; la diiodofluorescéine connu sous le nom CTFA CI 45425 ou Orange 10 ; la dibromofluorescéine connu sous le nom CTFA CI 45370 ou Orange 5 ; le sel de sodium de la tétrabromofluoroscéine connu sous le nom CTFA CI 45380 (Na salt) ou Red 22 ; le sel de sodium de la phloxine B connu sous le nom CTFA CI 45410 (Na salt) ou Red 28 ; le sel de sodium de la diiodofluorescéine connu sous le nom CTFA CI 45425 (Na salt) ou Orange 11 ; l'érythrosine connu sous le nom CTFA CI 45430 ou Acid Red 51 ; et la phloxine connu sous le nom CTFA CI 45405 ou Acid Red 98.

Comme activateur de la mélanogénèse, on peut notamment citer ceux décrits dans les demandes EP 1385472 et FR 2831439, et plus particulièrement des analogues de substrats de la tyrosinase, enzyme clé de la mélanogénèse tels que la tyrosine, la L-dopa ou la L-dihydroxylphenylalanine ; des activateurs de l'activité ou de l'expression de la tyrosinase tels que la forskoline, les bases xanthiques (théophylline, caféine), les peptides pro-opiomélanocortiques (ACTH, alpha-MSH ou autres agonistes de des récepteurs MC1), les diacylglycérols, les diols aliphatiques ou cycliques, les psoralènes, les prostaglandines et analogues, les activateurs de la proteinekinase G NO/cGMP dépendante ; des activateurs de transfert des mélanosomes vers les kératinocytes tels que les sérine protéases ou d'agonistes des récepteurs PAR-2 ; ou encore un extrait de Burnet (Sanguisorba officinalis) ou un extrait d'au moins un végétal du genre Chrysanthemum, particulièrement de l'espèce Chrysanthemum sinensis.

Le ou les agents de colorations peuvent être mis en oeuvre dans le procédé de l'invention, en mélange avec un ou plusieurs adjuvants solides ou pâteux, et de préférence pulvérulents. Les adjuvants peuvent être choisis parmi les argiles, les sels, les tensioactifs anioniques, non ioniques, cationiques ou zwittérioniques, les épaississants naturels ou de synthèse, l'amidon éventuellement modifié, les billes de verre, la silice, le nylon, l'alumine, le dioxyde de titane, les zéolithes, le poly(méthacrylate de méthyle) (PMMA), le chitosane, la maltodextrine, la cyclodextrine, les mono- ou disaccharides comme le glucose, le saccharose, le sorbitol ou le fructose, l'oxyde de zinc, de zirconium, les silicabades, le talc, l'acide polyaspartique, les borosilicates notamment de calcium, le polyéthylène, le coton, le polytétrafluoroéthylène (PTFE), la cellulose et ses dérivés, les composés superabsorbants, les carbonates de magnésium ou de calcium, les grains de maïs, le polyacrylamide, l'hydroxyapatite poreux, la soie, le collagène, la sciure de bois, la poudre de fucus, les farines ou extraits de blé, de riz, de pois, de lupin, de soja ou d'orge, la polyvinylpyrrolidone réticulée, l'alginate de calcium, le charbon actif, les particules de poly(chlorure de vinylidène/acrylonitrile), notamment celles commercialisées sous la dénomination générale d'« Expancel® » par la société AKZO NOBEL sous les références particulières « Expancel® WE» ou « DE » Expancels, et leurs mélanges.

Lorsqu'un ou plusieurs adjuvants sont présents, le ou les agents de coloration utilisés dans l'invention sont présents de préférence en une quantité allant de 0,5 à 99% en poids, mieux encore de 1 à 80 % en poids, et encore plus préférentiellement de 2 à 60 % en poids par rapport au poids total agent(s) de coloration et adjuvant(s) sous forme solide ou pâteuse.

Lorsque des plantes ou extraits de plantes sont utilisés dans le procédé selon l'invention, ils peuvent être soumis, avant la percolation, à un traitement tel qu'une torréfaction, un cryobroyage, ou une lyophilisation.

La composition de coloration permanente ou semi-permanente obtenue selon le procédé de l'invention contient outre le ou les agents de coloration et le(s) composant(s) du fluide, à savoir l'eau et/ou le(s) solvant(s) cosmétiquement acceptable(s), éventuellement tout ou partie du ou des adjuvants présents dans le mélange sous forme solide ou pâteuse.

L'invention concerne également une composition susceptible d'être obtenue par le procédé selon l'invention, la composition particulièrement préférée étant exempte d'agents conservateurs.

A partir du procédé de préparation de l'invention, on obtient une composition de coloration permanente ou semi-permanente de la peau, des paupières, des lèvres ou des ongles, de préférence de la peau, des paupières ou des lèvres, qui peut être appliquée directement sur la peau, les paupières, les lèvres ou les ongles, de préférence sur la peau, les paupières ou les lèvres, ou qui peut être mélangée à un milieu approprié à la coloration, ou encore au moins un additif classiquement utilisé en cosmétique pourra y être ajouté par un opérateur. On peut également mélanger au moins deux compositions obtenues par le procédé de l'invention. La composition de coloration de la peau, des paupières, des lèvres ou des ongles résultant éventuellement de mélange(s) et/ou addition(s) indiqués ci-dessus, sera dénommée ci-après composition finale de coloration ou composition finale.

Un mode de réalisation particulier de l'invention consiste à appliquer la composition obtenue, notamment sur la peau, par l'intermédiaire d'un dispositif ne nécessitant pas d'intervention humaine et équipé éventuellement d'un moyen de refroidissement.

Un autre mode de réalisation particulier consiste à ingérer la composition de coloration permanente ou semi-permanente obtenue selon le procédé de l'invention lorsque aucun problème de toxicité n'est connue dans le technique, par exemple lorsque l'agent de coloration est un caroténoïde, par exemple la β-carotène.

La quantité de ou des agents de coloration présents dans la composition finale de coloration est en général comprise dans l'intervalle allant de 0,001 à 50 % en poids environ, de préférence de 0,005 à 30 % en poids, et encore plus préférentiellement de 0,01 à 20% en poids par rapport au poids total de la composition finale de coloration.

Dans le cas où la composition de coloration permanente ou semi-permanente de la peau, des paupières, des lèvres ou des ongles obtenue par le procédé de la présente invention est mélangée à un milieu approprié pour la coloration, le milieu est généralement constitué par de l'eau ou par un mélange d'eau et d'au moins un solvant organique pour solubiliser les composés qui ne seraient pas suffisamment solubles dans l'eau. A titre de solvant organique, on peut citer, par exemple, les alcools inférieurs en C₁-C₄ tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants sont, de préférence, présents dans des proportions de préférence comprises entre 1 et 40% en poids par rapport au poids total de la composition finale, et encore plus préférentiellement entre 5 et 30% en poids.

Au moins un additif classiquement utilisé en cosmétique peut être également ajouté dans les compositions de coloration permanente ou semi-permanente de la peau, des paupières, des lèvres ou des ongles obtenues selon le procédé de la présente invention. A titre d'exemples de tels additifs, on peut citer des agents tensioactifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement, des agents filmogènes, des céramides, des agents conservateurs, des agents opacifiants.

Les additifs ci-dessus sont en général présents en quantité comprise pour chacun d'eux entre 0,01 et 20% en poids par rapport au poids de la composition finale.

Bien entendu, l'homme de l'art veillera à choisir ce ou ces éventuels composés de manière telle que les propriétés avantageuses attachées intrinsèquement à la composition coloration conforme à l'invention ne soient pas, ou substantiellement pas, altérées par la ou les adjonctions envisagées.

Le pH de la composition finale est généralement compris entre 3 et 12, et de préférence entre 5 et 11. Il peut être ajusté à la valeur désirée au moyen d'agents acidifiants ou alcalinisants habituellement utilisés en cosmétique ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les sels alcalinisants, on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule suivante : 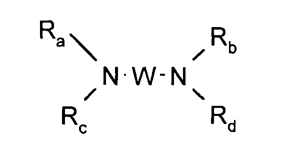 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

La composition finale de coloration de la peau, des paupières, des lèvres ou des ongles peut se présenter sous des formes diverses, telles que sous forme de liquides, de crèmes, de gels ou sous toute autre forme appropriée pour réaliser une coloration de la peau, des paupières, des lèvres ou des ongles.

La présente invention concerne aussi un procédé de coloration permanente ou semi-permanente de la peau, des paupières, des lèvres ou des ongles, comprenant la préparation d'une composition de coloration de la peau, des paupières, des lèvres ou des ongles par le procédé selon l'invention, et son application sur la peau, des paupières, des lèvres ou les ongles, par exemple par l'intermédiaire d'un opérateur ou par l'intermédiaire d'un dispositif ne nécessitant pas d'intervention humaine. La durée d'application peut varier entre 15 secondes et 1 heure.

Avant application, la composition de coloration permanente ou semi-permanente obtenue selon le procédé de l'invention, peut être mélangée à un milieu approprié pour la coloration et/ou à un ou plusieurs additifs classiquement utilisés en cosmétique tels que décrits ci-dessus.

Un autre mode de réalisation consiste à préparer au moins deux compositions de coloration permanente ou semi-permanente selon le procédé de l'invention, à les mélanger, et à ajouter éventuellement un milieu approprié pour la coloration et/ou un ou plusieurs additifs classiquement utilisés en cosmétique tels que décrits ci-dessus, puis à appliquer la composition finale obtenue sur la peau, les paupières, les lèvres ou les ongles.

Les exemples ci-après sont destinés à illustrer la présente invention.

### Exemple 1

On mélange les ingrédients suivants, sous forme de poudre :

| | |
|---|---|
| Dihydroxyacétone | 50 % en poids |
| Maltodextrine | 50 % en poids, |

par rapport au poids total du mélange pulvérulent.

On place ce mélange qui se trouve dans un récipient prévu pour recevoir un composé solide, dans une machine expresso du commerce. Ce mélange pulvérulent est ensuite traversé par la vapeur d'eau produite par la machine. On obtient ainsi une composition autobronzante prête à être appliquée sur la peau.

### Exemple 2

On fait passer de la vapeur d'eau selon le même mode opératoire que celui de l'exemple 1, sur une poudre de dihydroxyacétone qui se trouve dans le récipient prévu pour recevoir un composé solide. On obtient ainsi une composition autobronzante prête à être appliquée sur la peau.

## Revendications

1. Procédé de préparation d'une composition de coloration permanente ou semi-permanente de la peau, des paupières, des lèvres ou des ongles, **caractérisé en ce qu'**il comprend une étape de percolation de fluide sous une pression d'au moins 3 bars au travers d'au moins un agent de coloration sous forme solide ou pâteuse.

2. Procédé selon la revendication 1, **caractérisé en ce que** le fluide est constitué par de la vapeur d'eau éventuellement accompagnée d'eau liquide, ou par un ou plusieurs solvants liquides et/ou gazeux cosmétiquement acceptables, ou encore par un mélange de vapeur d'eau éventuellement accompagnée d'eau liquide, et d'un ou plusieurs solvants liquides et/ou gazeux cosmétiquement acceptables.

3. Procédé selon la revendication 2, **caractérisé en ce que** le fluide comprend au moins de la vapeur d'eau.

4. Procédé de préparation selon l'une quelconque des revendications, **caractérisé en ce que** l'agent de coloration est choisi parmi les autobronzants, les composés indoliques, les extraits végétaux colorants, les sels de flavylium non substitués en position 3 et substitués par au moins un groupe hydroxy ou alcoxy, les précurseurs de colorant du type polyphénol, les colorants naturels, le carmin de cochenille, les fluoranes ou leurs sels de métal alcalin, et les activateurs de la mélanogénèse.

5. Procédé selon la revendication 4, **caractérisé en ce que** les autobronzants sont des composés mono- ou polycarbonylés.

6. Procédé selon la revendication 5, **caractérisé en ce que** les autobronzants sont choisis parmi l'isatine, l'alloxane, la ninhydrine, le glycéraldéhyde, l'aldéhyde mésotartrique, la glutaraldéhyde, l'érythrulose, les dérivés de pyrazolin-4,5-diones, la dihydroxyacétone (DHA) et les dérivés de 4,4-dihydroxypyrazolin-5-ones.

7. Procédé selon la revendication 6, **caractérisé en ce que** l'autobronzant est la dihydroxyacétone.

8. Procédé selon la revendication 4, **caractérisé en ce que** les composés indoliques sont choisis parmi les monohydroxyindoles et les dihydroxyindoles.

9. Procédé selon la revendication 8, **caractérisé en ce que** le composé indolique est le 5,6-dihydroxyindole.

10. Procédé selon la revendication 4, **caractérisé en ce que** les extraits végétaux colorants sont choisis parmi les extraits végétaux du genre Sorgho.

11. Procédé selon la revendication 10, **caractérisé en ce que** les extraits végétaux colorants sont choisis parmi le Sorghum caudatum et le Sorghum Bicolor.

12. Procédé selon la revendication 4, **caractérisé en ce que** les sels de flavylium non substitués en position 3 et substitués par au moins un groupe hydroxy ou alcoxy sont choisis parmi le chlorure d'apigéninidine et le chlorure de 3', 4', 7- trihydroxyflavylium.

13. Procédé selon la revendication 4, **caractérisé en ce que** les précurseurs de colorant du type polyphénol sont des orthodiphénols.

14. Procédé selon la revendication 4, **caractérisé en ce que** les colorants naturels sont choisis parmi les flavonoïdes et leurs esters, éthers, hétérosides et polymères, les polyphénols non flavonoïdes et leurs esters, éthers, hétérosides et polymères, les caroténoïdes et β-laïnes, les chlorophylles ; les hydrates de carbone ; et les mélanines.

15. Procédé selon l'une quelconque des revendications précédentes, **caractérisé en ce que** dans lequel l'agent de coloration sous forme solide ou pâteuse est mis en oeuvre en mélange avec au moins un adjuvant.

16. Procédé selon la revendication 15, **caractérisé en ce que** le l'adjuvant est choisi parmi les argiles, les sels, les tensioactifs anioniques, non ioniques, cationiques ou zwittérioniques, les épaississants naturels ou de synthèse, l'amidon éventuellement modifié, les billes de verre, la silice, le nylon, l'alumine, le dioxyde de titane, les zéolithes, le poly(méthacrylate de méthyle) (PMMA), le chitosane, la maltodextrine, la cyclodextrine, les mono- ou disaccharides, l'oxyde de zinc, de zirconium, les silicabades, le talc, l'acide polyaspartique, les borosilicates notamment de calcium, le polyéthylène, le coton, le polytétrafluoroéthylène (PTFE), la cellulose et ses dérivés, les composés superabsorbants, les carbonates de magnésium ou de calcium, les grains de maïs, le polyacrylamide, l'hydroxyapatite poreux, la soie, le collagène, la sciure de bois, la poudre de fucus, les farines ou extraits de blé, de riz, de pois, de lupin, de soja ou d'orge, la polyvinylpyrrolidone réticulée, l'alginate de calcium, le charbon actif, et les particules de poly(chlorure de vinylidène/acrylonitrile).

17. Procédé selon la revendication 15 ou 16, **caractérisé en ce que** l'agent de coloration est présent en une quantité allant de 0,5 à 99% en poids, de préférence de 1 à 80 % en poids par rapport au poids total agent(s) de coloration et adjuvant(s) sous forme solide ou pâteuse.

18. Procédé selon l'une quelconque des revendications 15 à 17, **caractérisé en ce que** la composition de coloration obtenue contient outre le ou les agents de coloration et le(s) composant(s) du fluide, éventuellement tout ou partie du ou des adjuvants présents dans le mélange sous forme solide ou pâteuse.

19. Procédé selon l'une quelconque des revendications précédentes, **caractérisé en ce que** l'étape de percolation est mise en oeuvre avec un fluide sous pression de 3 à 30 bars.

20. Procédé selon la revendication 19, **caractérisé en ce que** l'étape de percolation est mise en oeuvre avec un fluide sous pression d'au moins 10 bars.

21. Composition de coloration permanente ou semi-permanente de la peau, des paupières, des lèvres ou des ongles susceptible d'être obtenue par le procédé selon l'une quelconque des revendications précédentes.

22. Composition de coloration permanente ou semi-permanente de la peau, des paupières, des lèvres ou des ongles selon la revendication 21, ne comprenant pas de conservateur.

23. Utilisation de la composition obtenue selon le procédé de l'une quelconque des revendications 1 à 20, pour la coloration permanente ou semi-permanente de la peau, des paupières, des lèvres ou des ongles.

24. Procédé de coloration permanente ou semi-permanente de la peau, des paupières, des lèvres ou des ongles, **caractérisé en ce que** l'on prépare une composition de coloration permanente ou semi-permanente selon le procédé selon l'une quelconque des revendications 1 à 20 et que l'on applique cette composition sur la peau, les paupières, les lèvres ou les ongles.

25. Procédé de coloration permanente ou semi-permanente selon la revendication 24, **caractérisé en ce que** l'on applique cette composition sur la peau par l'intermédiaire d'un dispositif ne nécessitant pas d'intervention humaine.

26. Procédé de coloration permanente ou semi-permanente selon la revendication 24, **caractérisé en ce que**, avant l'application, la composition de coloration préparée selon le procédé selon l'une quelconque des revendications 1 à 20, est mélangée à un milieu approprié pour la coloration et/ou à un ou plusieurs additifs utilisés en cosmétique.

27. Procédé de coloration permanente ou semi-permanente de la peau, des paupières, des lèvres ou des ongles, **caractérisé en ce que** l'on prépare au moins deux compositions de coloration permanente ou semi-permanente selon le procédé selon l'une quelconque des revendications 1 à 20, on les mélange et on applique le mélange sur la peau, les paupières, les lèvres ou les ongles.

28. Dispositif de conditionnement d'une composition cosmétique, comprenant un logement fermé délimité par au moins une paroi au moins en partie perméable à de la vapeur d'eau sous pression d'au moins 3 bars, ladite composition cosmétique contenant au moins un agent de coloration sous forme solide ou pâteuse.

29. Dispositif selon la revendication 28, **caractérisé en ce que** le logement est délimité par deux feuilles scellées.

30. Dispositif selon la revendication 28, **caractérisé en ce que** le logement est délimité par une barquette fermée par un couvercle.
